# EUROPÄISCHE PATENTSCHRIFT

(11) **EP 2 782 607 B1**
(45) Veröffentlichungstag und Bekanntmachung des Hinweises auf die Patenterteilung: **22.09.2021**
(21) Anmeldenummer: 12794927.9
(22) Anmeldetag: 23.11.2012
(51) Int. Cl.: A61L 27/18, A61L 27/54, C08L 71/02

(54) **SYNTHETISCHES SCHMIERMITTEL ZUR VERWENDUNG ALS SYNOVIALFLÜSSIGKEITSERSATZ**
SYNTHETIC LUBRICANT FOR USE AS A SYNOVIAL FLUID REPLACEMENT
LUBRIFIANT SYNTHÉTIQUE S'UTILISANT COMME SUBSTITUT DU LIQUIDE SYNOVIAL

(30) Priorität: 24.11.2011 DE 102011055683
(43) Veröffentlichungstag der Anmeldung: 01.10.2014
(73) Patentinhaber: von Foerster, Götz, 20249 Hamburg (DE)
(72) Erfinder: von Foerster, Götz, 20249 Hamburg (DE)
(74) Vertreter: von dem Borne, Andreas
(86) Internationale Anmeldenummer: PCT/EP2012/073520
(87) Internationale Veröffentlichungsnummer: WO 2013/076271

(56) Entgegenhaltungen:
- WO-A1-00/48636
- WO-A1-2005/099669
- WO-A2-97/33563
- DE-A1- 2 313 221
- GB-A- 2 268 507
- US-A- 4 141 973
- "PERFLUORPOLYETHER, FOMBLIN Y, M und Z Öle + Fomblin Fette", SOLVAY SOLEXIS , Bd. Ausgabe 01/04 2004, Seiten 1-23, XP002690778, Gefunden im Internet: URL:http://images.mascom-bremen.de/info_FO MBLIN_PFPE_Oele_und_Fette.pdf [gefunden am 2013-01-22]
- "Medical Devices: Guidance document", , 3 December 2009 (2009-12-03), XP055023202, Retrieved from the Internet: URL:http://ec.europa.eu/health/medical-dev ices/files/meddev/2_1_3_rev_3-12_2009_en.p df [retrieved on 2012-03-28]

## Beschreibung

Die Erfindung betrifft ein flüssiges, synthetisches Schmiermittel, welches aus zumindest einem Perfluorpolyether (PFPE) besteht, zur Verwendung als Synovialflüssigkeitsersatz für ein erkranktes natürliches Gelenk eines menschlichen oder tierischen Körpers, wobei ein Perfluorpolyether gemäß Anspruch 1 verwendet wird. - Bei dem Gelenk handelt es sich vorzugsweise um ein Hüftgelenk oder Kniegelenk, alternativ werden jedoch auch andere Gelenke umfasst, zum Beispiel Schultergelenke.

In den Gelenken menschlicher oder tierischer Körper wird eine natürliche Gelenkflüssigkeit produziert, die auch als Synovia oder Synovialflüssigkeit bezeichnet wird. Es handelt sich um eine viskose, klare Körperflüssigkeit, die insbesondere der Versorgung des Gelenkkörpers und der Schmierung der Gelenkflächen dient. Außerdem trägt sie gemeinsam mit dem Gelenkknorpel zur Stoßdämpfung innerhalb der Gelenke bei. Bei krankhaften Veränderungen des Gelenkes, zum Beispiel durch Verletzungen, entzündliche Erkrankungen oder auch durch Verschleißvorgänge kann es auch zu einer krankhaften Veränderung der Gelenkflüssigkeit selbst kommen. So tritt bei einem arthrotischen Gelenk häufig nicht nur eine Schädigung des Knorpels selbst, sondern auch eine Schädigung der Gelenkflüssigkeit auf. Es besteht daher grundsätzlich das Bedürfnis, einen Ersatz für die körpereigene Synovialflüssigkeit zur Verfügung zu stellen.

So wurden bereits Therapien entwickelt, bei denen die krankhaft veränderte Gelenkflüssigkeit durch eine künstliche Gelenkflüssigkeit ersetzt wird, zum Beispiel im Zuge der Arthrosebehandlung. Als Gelenkflüssigkeitsersatz kommt dabei künstlich hergestellte Hyaluronsäure zum Einsatz. Grundsätzlich ist Hyaluronsäure ein körpereigener Stoff, der auch in der natürlichen Synovialflüssigkeit der Gelenke auftritt. Hyaluronsäure lässt sich grundsätzlich künstlich herstellen. Die Herstellung ist jedoch verhältnismäßig aufwendig.

Alternativ wurde in der Praxis ein Synovialflüssigkeitsersatz auf Basis einer Polytetrafluorethylen-Emulsion ("Teflon-Emulsion") vorgeschlagen. Diese hat jedoch den Nachteil, dass sie mit menschlichem Gewebe reagiert, so dass der Einsatz langfristig nicht zu dem gewünschten Erfolg führt.

Es besteht daher grundsätzlich das Bedürfnis, einen nicht biodegradierbaren Synovialflüssigkeitsersatz zur Verfügung zu stellen, der sich wirtschaftlich künstlich herstellen lässt und zugleich durch hervorragende Eigenschaften auszeichnet. - Hier setzt die Erfindung ein.

Der Erfindung liegt die Aufgabe zugrunde, einen Synovialflüssigkeitsersatz zu schaffen, der nicht biodegradierbar ist und sich wirtschaftlich herstellen lässt.

Zur Lösung dieser Aufgabe lehrt die Erfindung ein flüssiges, synthetisches Schmiermittel, welches aus zumindest einem Perfluorpolyether (PFPE, auch: perfluorierte Polyether) besteht, zur Verwendung als Synovialflüssigkeitsersatz für ein erkranktes natürliches Gelenk eines menschlichen oder tierischen Körpers, wobei ein Perfluorpolyether gemäß Anspruch 1 verwendet wird.

Ein solches flüssiges, synthetisches Schmiermittel aus Perfluorpolyether ist grundsätzlich bekannt. Es wird zum Beispiel von der italienischen Firma Solvay Solexis unter dem Handelsnamen FOMBLIN vertrieben. Perfluorpolyether setzen sich ausschließlich aus den Elementen Fluor, Kohlenstoff und Sauerstoff zusammen. Durch die stabile atomare Bindung vom Fluor zum Kohlenstoff zeichnen sich Perfluorpolyether durch hervorragendes inertes Verhalten aus. Schmiermittel aus Perfluorpolyether werden daher seit langem als hochwertige technische Schmiermittel für verschiedenste technologische Anwendungen eingesetzt.

So beschreibt die EP 0 244 838 A1 die Verwendung eines Perfluorpolyethers mit linearer Struktur mit einer kinematischen Viskosität zwischen 8.000 und 40.000 cSt bei 20 °C als technisches Schmiermittel.

In der DE 10 2006 035 551 A1 wird die Verwendung von perfluoriertem Polyether als Gleitmittel im Zusammenhang mit magnetischen Plattenlaufwerken (Festplatten) beschrieben.

Die DE 10 2009 041 132 A1 beschreibt ein pharmazeutisches Packmittel mit Gleitfilm, welches von einem Fluid mit fluorierten oder perfluorierten Polyethern gebildet wird.

Der Einsatz von synthetischen, fluorierten Ölen wird auch im Zusammenhang mit Schmierölen für Wälzlager von medizinischen Geräten, zum Beispiel zahnärztlichen Luftturbinen-Handstücken, beschrieben (vgl. DE 197 43 041 B4).

Im Übrigen wurde im Zusammenhang mit der Herstellung von Kontaktlinsen beschrieben, für das Linsenmaterial Polysiloxane mit perfluorierten Ethereinheiten einzusetzen (vgl. zum Beispiel DE 296 24 309 U1).

In der WO 97/33563 A1 wird die Verwendung von Fluorkohlenwasserstoff zur medizinischen Diagnose und Behandlung von Gelenkerkrankungen beschrieben.

Die US 2004/0217324 A1 beschreibt eine magnetorheologische Flüssigkeit zur Verwendung in einem künstlichen Gelenk, wobei die magnetorheologische Flüssigkeit polarisierbare Eisenpartikel, eine Trägerflüssigkeit und optional Additive enthält. Die Trägerflüssigkeit kann einen perfluorierten Polyether enthalten. Die magnetorheologische Flüssigkeit wird vollständig in einem künstlichen Gelenk gekapselt.

Die Erfindung hat nun erkannt, dass sich ein flüssiges, synthetisches Schmiermittel, welches aus Perfluorpolyether bzw. aus perfluoriertem Polyether gemäß Anspruch 1 besteht, hervorragend als nicht biodegradierbarer Synovialflüssigkeitsersatz verwenden lässt. Dieses Schmiermittel ist chemisch inert, nicht toxisch und biologisch absolut inaktiv. Das Schmiermittel lässt sich mit verschiedensten Viskositäten herstellen, so dass eine optimale Anpassung an die gewünschten Viskositätswerte des Synovialflüssigkeitsersatzes möglich ist. Das Schmiermittel zeichnet sich durch einen niedrigen Dampfdruck und hohe Benetzbarkeit aus. Der erfindungsgemäße Synovialflüssigkeitsersatz lässt sich in herkömmlicher Weise applizieren bzw. verarbeiten. Bevorzugt wird der erfindungsgemäße Synovialflüssigkeitsersatz in das zu behandelnde Gelenk, zum Beispiel Hüftgelenk, Kniegelenk oder dergleichen, injiziert, das heißt durch intraartikuläre Injektion.

Das Schmiermittel wird bei erkrankten, natürlichen Gelenken eines menschlichen oder tierischen Körpers eingesetzt. Es handelt es sich folglich um ein Implantat. Alternativ (nicht erfindungsgemäß) wird das Schmiermittel bei künstlichen Gelenken eingesetzt, zum Beispiel um deren Abrieb zu verringern.

Das erfindungsgemäße Schmiermittel, welches aus Perfluorpolyether beziehungsweise Perfluorpolyethern besteht, ist biokompatibel nach ISO 10993.

Besonders vorteilhaft ist dabei die Tatsache, dass das Material omniphob ist. Dieses meint im Rahmen der Erfindung, dass sich das Schmiermittel nicht wie andere Flüssigkeiten in Tropfen verteilt, sondern selbstständig zusammen zieht. Das Material ist gegen alle (anderen) Flüssigkeiten abstoßend, und zwar sowohl gegen hydrophobe Flüssigkeiten als auch gegen hydrophile Flüssigkeiten. Das Schmiermittel aus PFPE reorganisiert sich stets selbst, und zwar in vivo. Dadurch bildet sich permanent ein neuer Gleitfilm oberflächlich aus. Das Schmiermittel verteilt sich folglich nicht einfach nur (wie bei sehr zähflüssigen bzw. halbfesten omniphoben Materialien) nach Beanspruchung, sondern es organisiert sich nach jeder Beanspruchung neu. Die Neuorganisation führt zu einer permanenten Neuausbildung der homogenen Gleitschicht in situ. Diese selbstorganisierte Gleitschicht hat keine Unterbrechung und ist bei ausreichender Einsatzmenge flächig.

Das erfindungsgemäße Schmiermittel aus Perfluorpolyether liegt zumindest bei einer Temperatur von 37 °C als Flüssigkeit vor.

Erfindungsgemäß wird ein Perfluorpolyether mit inerten Endgruppen R, R' verwendet, wobei die Endgruppen R, R' jeweils aus der Gruppe "Trifluormethylgruppe (-CF₃), Pentafluorethylgruppe (-C₂F₅), Trifluormethoxygruppe (-OCF₃), Pentafluorethoxygruppe (-OC₂F₅)" ausgewählt sind. In einer besonders bevorzugten Ausführungsform ist eine Endgruppe R eine Trifluormethylgruppe (-CF₃) und eine andere Endgruppe R' eine Trifluormethoxygrupppe (-OCF₃).

Es wird ein Perfluorpolyether mit verzweigter, sternförmiger oder linearer Struktur verwendet. Gemäß einer Ausführungsform wird ein Perfluorpolyether mit der chemischen Struktur R-(O-CFR"-CF₂-)ₙ (O-CF₂)ₘ-R' verwendet, wobei R" eine Fluoralkylgruppe mit der Formel CₐF₂ₐ₊₁ mit 1 ≤ a ≤ 10 ist. Dabei ist n>1 und m≥1. Besonders bevorzugt ist die funktionelle Gruppe R" eine Trifluormethylgruppe. Das Verhältnis n/m kann vorzugsweise 20 bis 40 betragen. Bei dieser Ausführungsform kann es sich zum Beispiel um einen Perfluorpolyether vom Typ "FOMBLIN Y" der Firma Solvay Solexis handeln. Auch dieser Perfluorpolyether zeichnet sich durch eine äußerst stabile, kovalente C-F-Bindung aus. Die sterisch anspruchsvollen und gleichförmig auf ihrer Oberfläche polarisierten, vorzugsweise perfluorierten Seitenketten schützen die C-C-Kette des Polymerrückgrats vor thermischen Einflüssen und Schirmen das Polymerrückgrat gegen chemische Angriffe ab.

Alternativ wird ein Perfluorpolyether mit linearer Struktur verwendet, zum Beispiel vom Typ FOMBLIN M, FOMBLIN W oder FOMBLIN Z. Dabei wird ein Perfluorpolyether mit der folgenden linearen Struktur verwendet: R-(-O-CFR"-CF₂-)ₚ (O-CF₂)_{q}-R', wobei R" ein Fluoratom ist. Das Verhältnis p/q kann zum Beispiel den Wert 0,8 einnehmen. Dabei ist p>1 und q>1. Auch diese flüssigen Polymere bestehen ausschließlich aus Kohlenstoff, Fluor und Sauerstoffatomen. Sie weisen jedoch keine Seitengruppen auf.

Es ist möglich, dass ein aus mindestens einem der perfluorierten Polyether bestehendes Polymernetzwerk eingesetzt bzw. verwendet wird. Vorteilhafterweise besteht das Polymernetzwerk aus den miteinander vernetzten, linearen und/oder verzweigten Perfluorpolyethern. Es hat sich bewährt, dass die Perfluorpolyether aktiviert und vorzugsweise (nicht erfindungsgemäß) durch das Aufpfropfen von funktionellen Gruppen, beispielsweise Maleinsäurederivaten, aktiviert sind. Es liegt in diesem Rahmen, dass die aktivierten Perfluorpolyether über ihre funktionellen Gruppen miteinander vernetzt sind. Gemäß einer Ausführungsform bestehen die Polymernetzwerke ausschließlich aus perfluorierten Polyethern bzw. Perfluorpolyethern.

Von besonderer Bedeutung ist darüber hinaus, dass das Schmiermittel aus PFPE spritzbar und folglich injizierbar ist.

Durch besonders gute Eigenschaften zeichnet sich der erfindungsgemäße Synovialflüssigkeitsersatz aus, wenn er eine kinematische Viskosität (bei 20 °C) von 50 bis 1000 cSt aufweist, vorzugsweise 100 bis 500 cSt, besonders bevorzugt 200 bis 300 cSt. Diese Angaben können sich zum Beispiel auf die Bestimmungsmethode gemäß ASTM D 445 beziehen. Bei einer Temperatur von 40 °C liegt die kinematische Viskosität zum Beispiel bei 200 bis 300 cSt, vorzugsweise 50 bis 150 cSt, besonders bevorzugt 70 bis 100 cSt.

Die mittlere Molekülmasse (das heißt das mittlere Molekulargewicht) kann zumindest 1000 u, zum Beispiel 1000 bis 5000 u, vorzugsweise 2000 bis 4000 u, betragen. Bevorzugt wird ein Perfluorpolyether mit einer Dichte von 1,5 bis 2,5, vorzugsweise 1,7 bis 2,0, besonders bevorzugt 1,8 bis 1,95 g/cm³ verwendet, und zwar bezogen auf eine Temperatur von 20 °C.

Insgesamt besteht die Möglichkeit, den nicht biodegradierbaren Synovialflüssigkeitsersatz aus Perfluorpolyether hinsichtlich seiner Eigenschaften optimal an die Eigenschaften, zum Beispiel die Viskosität, natürlicher Synovialflüssigkeit anzupassen.

Nach einem weiteren Vorschlag liegt es im Rahmen der Erfindung, dem Schmiermittel bzw. dem Perfluorpolyether, Additive als pharmazeutische Wirkstoffe zuzusetzen. So können zum Beispiel pharmazeutische Wirkstoffe in dem Perfluorpolyether suspendiert werden. Auf diese Weise besteht die Möglichkeit, dem Synovialflüssigkeitsersatz zum Beispiel Antibiotika als Infektionsschutz oder dergleichen beizufügen. Die Tatsache, dass das Schmiermittel als Synovialflüssigkeitsersatz (ausschließlich) aus Perfluorpolyether besteht, schließt folglich nicht aus, dass Additive als pharmazeutische Wirkstoffe zugesetzt bzw. mit appliziert werden. Dann wird folglich eine Suspension appliziert, die im Wesentlichen aus dem Schmiermittel und zusätzlich dem pharmazeutischem Wirkstoff besteht.

Insgesamt schlägt die Erfindung ein Schmiermittel der beschriebenen Art, welches folglich aus zumindest einem der Polyfluorpolyether (PFPE) des Anspruchs 1 besteht, zur Verwendung als Synovialflüssigkeitsersatz für ein erkranktes, natürliches Gelenk eines menschlichen oder tierischen Körpers vor. Der Verwendung der Perfluorpolyether (PFPE) zur Herstellung eines Schmiermittels als Synovialflüssigkeitsersatz kommt folglich besondere Bedeutung zu.

Folgendes bevorzugtes Ausführungsbeispiel kann eingesetzt werden:
Es handelt sich um einen Synovialflüssigkeitsersatz aus einem Perfluorpolyether vom Typ FOMBLIN Y der Firma Solvay Solexis. Dabei kann bevorzugt der Typ FOMBLIN Y 25 eingesetzt werden, welcher ein mittleres Molekulargewicht von 3200 u und eine Dichte von 1,90 g/cm³ bei 20 °C aufweist. Die kinematische Viskosität beträgt bei 20 °C 250 cSt und bei 40 °C 81 cSt. Der Viskositätsindex beträgt 108. Die Oberflächenspannung liegt bei 22 dynes/cm.

Dieser Perfluorpolyether vom Typ FOMBLIN Y weist folgende chemische Struktur auf: CF₃-(O-C(CF₃)F-CF₂)n-(O-CF₂)m-O-CF₃, mit n > 1 und m ≥ 1 sowie mit n/m = 20-40.

## Patentansprüche

1. Flüssiges synthetisches Schmiermittel, welches aus zumindest einem Perfluorpolyether (PFPE) besteht, zur Verwendung als Synovialflüssigkeitsersatz für ein erkranktes natürliches Gelenk eines menschlichen oder tierischen Körpers,
wobei ein Perfluorpolyether mit inerten Endgruppen R, R' verwendet wird, wobei die Endgruppen R, R' jeweils aus der Gruppe "Trifluormethylgruppe (-CF₃), Pentafluorethylgruppe (-C₂F₅), Trifluormethoxygruppe (-OCF₃), Pentafluorethoxygruppe (-OC₂F₅)" ausgewählt sind,
wobei ein Perfluorpolyether mit verzweigter oder sternförmiger Struktur
R-(-O-CFR"-CF₂-)ₙ(O-CF₂)ₘ-R'
verwendet wird, wobei n > 1 und m ≥ 1 ist und wobei R" eine Fluoralkylgruppe mit der Formel CₐF₂ₐ₊₁ mit 1 ≤ a ≤ 10 ist,
und/oder
wobei ein Perfluorpolyether mit linearer Struktur
R-(-O-CF₂-CF₂-)ₚ(O-CF₂)_{q}-R'
verwendet wird, wobei p > 1 und q > 1 ist.

2. Schmiermittel nach Anspruch 1, wobei das Schmiermittel zumindest bei einer Temperatur von 37 °C als Flüssigkeit vorliegt.

3. Schmiermittel nach einem der Ansprüche 1 oder 2, wobei R" eine Trifluormethylgruppe ist und wobei das Verhältnis von n/m bevorzugt 20 bis 40 beträgt.

4. Schmiermittel nach einem der Ansprüche 1 bis 3, mit p/q ist = 0,8.

5. Schmiermittel nach einem der Ansprüche 1 bis 4, wobei ein Perfluorpolyether mit einer kinematischen Viskosität (bei 20 °C) von 50 bis 1000 cSt, vorzugsweise 100 bis 500 cSt, besonders bevorzugt 200 bis 300 cSt verwendet wird.

6. Schmiermittel nach einem der Ansprüche 1 bis 5, wobei ein Perfluorpolyether mit einer kinematischen Viskosität (bei 40 °C) von 20 bis 300 cSt, vorzugsweise 50 bis 150 cSt, besonders bevorzugt 70 bis 100 cSt verwendet wird.

7. Schmiermittel nach einem der Ansprüche 1 bis 6, wobei ein Perfluorpolyether mit einer Dichte von 1,5 bis 2,5, vorzugsweise 1,7 bis 2,0, besonders bevorzugt 1,8 bis 1,95 g/cm³ (bei 20 °C) verwendet wird.

8. Schmiermittel nach einem der Ansprüche 1 bis 7, wobei ein Perfluorpolyether mit einer mittleren Molekülmasse von 1000 u, zum Beispiel zumindest 2000 u, bevorzugt 1000 bis 5000 u, vorzugsweise 2000 bis 4000 u, verwendet wird.

9. Schmiermittel nach einem der Ansprüche 1 bis 8, wobei dem Schmiermittel bzw. dem Perfluorpolyether Additive als pharmazeutische Wirkstoffe zugesetzt sind, wobei bevorzugt pharmazeutische Wirkstoffe in dem perfluorierten Polyether suspendiert sind.

## Claims

1. A liquid, synthetic lubricant which consists of at least one perfluoropolyether (PFPE), for use as a synovial fluid replacement for a diseased natural joint of a human or animal body,
wherein a perfluoropolyether with inert end groups R, R' is used, wherein the end groups R, R' are respectively selected from the "trifluoromethyl group (-CF₃), pentafluoroethyl group (-C₂F₅), trifluoromethoxy group (-OCF₃), pentafluoroethoxy group (-OC₂F₅)" group,
wherein a perfluoropolyether with a branched or star-shaped structure
**R-(-O-CFR"-CF₂-)ₙ(O-CF₂)ₘ-R'**
is used, wherein n > 1 and m ≥ 1 and wherein R" is a fluoroalkyl group with the formula CₐF₂ₐ₊₁ in which 1 ≤ a ≤ 10,
and/or
wherein a perfluoropolyether with a linear structure
**R-(-O-CF₂-CF₂-)ₚ(O-CF₂)_{q}-R'**
is used, wherein p > 1 and q > 1.

2. The lubricant as claimed in claim 1, wherein the lubricant is a liquid at least at a temperature of 37°C.

3. The lubricant as claimed in claim 1 or claim 2, wherein R" is a trifluoromethyl group and wherein the ratio of n/m is preferably 20 to 40.

4. The lubricant as claimed in one of claims 1 to 3, in which p/q is equal to 0.8.

5. The lubricant as claimed in one of claims 1 to 4, wherein a perfluoropolyether with a kinematic viscosity (at 20°C) of 50 to 1000 cSt, preferably 100 to 500 cSt, particularly preferably 200 to 300 cSt, is used.

6. The lubricant as claimed in one of claims 1 to 5, wherein a perfluoropolyether with a kinematic viscosity (at 40°C) of 20 to 300 cSt, preferably 50 to 150 cSt, particularly preferably 70 to 100 cSt, is used.

7. The lubricant as claimed in one of claims 1 to 6, wherein a perfluoropolyether with a density of 1.5 to 2.5, preferably 1.7 to 2.0, particularly preferably 1.8 to 1.95 g/cm³ (at 20°C), is used.

8. The lubricant as claimed in one of claims 1 to 7, wherein a perfluoropolyether with an average molecular mass of 1000 u, for example at least 2000 u, preferably 1000 to 5000, advantageously 2000 to 4000 u, is used.

9. The lubricant as claimed in one of claims 1 to 8, wherein additives are added to the lubricant or the perfluoropolyether as pharmaceutical active substances, wherein preferably, the pharmaceutical active substances are suspended in the perfluorinated polyether.

## Revendications

1. Lubrifiant synthétique liquide, lequel est constitué d'au moins un perfluoropolyéther (PFPE), destiné à l'utilisation en tant que substitut de liquide synovial pour une articulation naturelle malade d'un corps humain ou animal,
un perfluoropolyéther à groupes terminaux inertes R, R' étant utilisé, les groupes terminaux R, R' étant respectivement sélectionnés dans le groupe « groupe trifluorométhyle (-CF₃), groupe pentafluoroéthyle (-C₂F₅), groupe trifluorométhoxy (-OCF₃), groupe pentafluoréthoxy (-OC₂F₅) »,
un perfluoropolyéther à structure ramifiée ou en forme d'étoile
**R-(-O-CFR"-CF₂-)ₙ(O-CF₂)ₘ-R'**
étant utilisé, n étant > 1 et m étant ≥ 1 et R" étant un groupe fluoroalkyle de formule CₐF₂ₐ₊₁ avec 1 ≤ a ≤ 10,
et/ou,
un perfluoropolyéther de structure linéaire
**R-(-O-CF₂-CF₂-)ₚ(O-CF₂)_{q}-R'**
étant utilisé, p étant > 1 et q étant > 1.

2. Lubrifiant selon la revendication 1, le lubrifiant se présentant sous la forme d'un liquide au moins à une température de 37 °C.

3. Lubrifiant selon l'une quelconque des revendications 1 ou 2, R" étant un groupe trifluorométhyle et le rapport n/m étant de préférence de 20 à 40.

4. Lubrifiant selon l'une quelconque des revendications 1 à 3, avec p/q est = 0,8.

5. Lubrifiant selon l'une quelconque des revendications 1 à 4, un perfluoropolyéther d'une viscosité cinématique (à 20 °C) de 50 à 1000 cSt, de préférence de 100 à 500 cSt, de manière particulièrement préférentielle, de 200 à 300 cSt étant utilisé.

6. Lubrifiant selon l'une quelconque des revendications 1 à 5, un perfluoropolyéther d'une viscosité cinématique (à 40 °C) de 20 à 300 cSt, de préférence de 50 à 150 cSt, de manière particulièrement préférentielle, de 70 à 100 cSt étant utilisé.

7. Lubrifiant selon l'une quelconque des revendications 1 à 6, un perfluoropolyéther d'une densité de 1,5 à 2,5, de préférence de 1,7 à 2,0, de manière particulièrement préférentielle, de 1,8 à 1,95 g/cm³ (à 20 °C) étant utilisé.

8. Lubrifiant selon l'une quelconque des revendications 1 à 7, un perfluoropolyéther d'une masse moléculaire moyenne de 1000 u, par exemple d'au moins 2000 u, préférentiellement de 1000 à 5000 u, de préférence de 2000 à 4000 u étant utilisé.

9. Lubrifiant selon l'une quelconque des revendications 1 à 8, au lubrifiant ou au perfluoropolyéther étant ajoutés des additifs en tant que principes actifs pharmaceutiques, des principes actifs pharmaceutiques étant de préférence en suspension dans le polyéther perfluoré.
